# EUROPEAN PATENT SPECIFICATION

(11) **EP 2 874 653 B1**
(45) Date of publication and mention of the grant of the patent: **27.12.2017**
(21) Application number: 13747541.4
(22) Date of filing: 11.07.2013
(51) Int. Cl.: A61K 39/02

(54) **METHOD OF PROVIDING PROTECTIVE IMMUNITY AGAINST HETEROLOGOUS LEPTOSPIRA STRAINS**
VERFAHREN ZUR HERSTELLUNG EINER SCHÜTZENDEN IMMUNITÄT GEGEN HETEROLOGE LEPTOSPIRA-STÄMME
PROCÉDÉ DE FOURNITURE D'UNE IMMUNITÉ PROTECTRICE CONTRE DES SOUCHES HÉTÉROLOGUES DE LEPTOSPIRA

(30) Priority: 17.07.2012 US 201261672386 P
(43) Date of publication of application: 27.05.2015
(73) Proprietor: Merial, Inc., Duluth, GA 30096 (US)
(72) Inventor: PARDO, Maria, Camila, Athens, GA 30605 (US)
(74) Representative: D Young & Co LLP
(86) International application number: PCT/US2013/049997
(87) International publication number: WO 2014/014725

(56) References cited:
- ROSARIO LUIS A ET AL: "Cross-protection among unrelated leptospira pathogens serovars: an unfinished story.", ADVANCES IN CLINICAL AND EXPERIMENTAL MEDICINE : OFFICIAL ORGAN WROCLAW MEDICAL UNIVERSITY 2012 SEP-OCT, vol. 21, no. 5, September 2012 (2012-09), pages 581-589, XP002716092, ISSN: 1899-5276
- GONZALEZ M ET AL: "Characterization of Leptospira Ballum strains isolated from clinical cases. Cross immunity in Hamsters vaccinated with vax-SPIRAL(R)", BIOTECNOLOGIA APLICADA 200404 CU, vol. 21, no. 2, April 2004 (2004-04), pages 77-81, XP008165285, ISSN: 0864-4551
- RINEHART CAROL L ET AL: "Efficacy of vaccination of cattle with the Leptospira interrogans serovar hardjo type hardjoprajitno component of a pentavalent Leptospira bacterin against experimental challenge with Leptospira borgpetersenii serovar hardjo type hardjo-bovis", AMERICAN JOURNAL OF VETERINARY RESEARCH : AJVR, AMERICAN VETERINARY MEDICAL ASSOCIATION. - CHICAGO, ILL. : ASSOC., 1940, US, vol. 73, no. 5, 1 May 2012 (2012-05-01), pages 735-740, XP008165071,
- SRIKRAM AMPORN ET AL: "Cross-protective Immunity Against Leptospirosis Elicited by a Live, Attenuated Lipopolysaccharide Mutant", JOURNAL OF INFECTIOUS DISEASES, vol. 203, no. 6, March 2011 (2011-03), pages 870-879, XP002716093, ISSN: 0022-1899

## Description

### INCORPORATION BY REFERENCE

This application claims priority to provisional application USSN 61/672,386, filed on July 17, 2012.

### FIELD OF THE INVENTION

The present invention relates generally to immunogenic leptospira compositions, which are capable of eliciting cross-protective immune responses in animals, particularly canine animals. The invention further relates to providing animals, especially canine animals, with cross-protective immune responses against leptospira.

### BACKGROUND OF THE INVENTION

Leptospirosis is an important world-wide zoonosis, caused by spirochetes from the Leptospira genus. It is an occupational hazard for many people who work outdoors or with animals, including farmers, veterinarians, meat workers, dairy farmers, and military personnel. It is a recreational hazard for campers, or those who participate in outdoor sports in contaminated areas, and has been associated with swimming, wading, and whitewater rafting. Outbreaks of leptospirosis are usually caused by exposure to water contaminated with the urine of infected animals. Many different kinds of animals carry the bacterium; they may become sick but sometimes have no symptoms. Leptospira organisms have been found in cattle, pigs, horses, dogs, rodents, and wild animals, including marine mammals. Humans become infected through contact with water, food, or soil containing urine from these infected animals. This may happen by swallowing contaminated food or water or through skin contact, especially with mucosal surfaces such as the eyes or nose, or with broken skin.

The most common serovars reported in the United States are *L. icterohaemorrhagiae, L. canicola*, *L. grippotyphosa, L. canicola* and *L. bratislava.* Another serovar of interest reported in Latin America, is *L. interrogans* serovar Copenhageni. This serovar belongs to the Icterohaemorrhagiae serogroup, and has similarities in the DNA sequence for known colonization virulence factors, and appears to be responsible for most canine leptospirosis in New Zealand.

### Review of the Literature

"Leptospirosis Fact Sheet" (WHO, Regional Office for South-East Asia, 2009) indicates, in part, that animals and humans can be immunized, but that protection is largely serovar-specific. Lack of cross-protection is not surprising, particularly in view of the significant genetic/genomic differences, for example, among the gene organization in the lipopolysaccharide biosynthetic (rfb) locus (Pena-Moctezuma, A. et al., 2001 FEMS Immunology and Medical Microbiology 31 (2001) 73-81).

Nascimento, A.L.T.O. et al. Comparative genomics of two Leptospira interrogans serovars reveals novel insights into physiology and pathogenesis. Journal of Bacteriology, 186(7):2164-2172, 2004.

Nascimento, A.L.T.O. et al. Genome features of Leptospira interrogans serovar Copenhageni. Braz J Med Biol Res. 2004 Apr; 37(2).

Recombitek Lepto 4 (Merial Limited) - contains Leptospira icterohaemorrhagiae (LI) was obtained from National Animal Disease Center (NADC), Ames, Iowa, on 28 February 1968 by Dow Chemical, Zionsville, Indiana. The vaccine further contains Leptospira grippotyphosa, L. canicola, and L. pomona serovars.

Novibac (Merck Animal Health) - contains L. interrogans serogroup Canicola serovar Portland-vere (strain Ca-12-000); L. interrogans serogroup Icterohaemorrhagiae serovar copenhageni (strain Ic-02-001); L. interrogans serogroup Australis serovar Bratislava (strain As-05-073); and L. kirschneri serogroup Grippotyphosa serovar Dadas (strain Gr-01-005). Company-provided data indicates, unsurprisingly, the Copenhageni-containing vaccine elicits in canine an immune response against serovar copenhageni. This product label claim is also consistent with what is well-known in the Leptospira arts, namely, that little if any evidence for "cross-protection", which is herein defined as providing protection against a heterologous Leptospira serovar by administering an effective amount of a different serovar (e.g. protecting against copenhageni by administering a homologous effective amount of a non-copenhageni LI Icterohaemorrhagiae serogroup Leptospire.

A pentavalent Leptospira vaccine containing L. interrogans serovar hardjo type hardjoprajitno provides cattle with protection against a challenge with L. borgpetersenii serovar hardjo type hardjo-bovis strain 203 (Rinehart, CL. et al., 2012 American Journal of Veterinary Research 73 (2012) 735-740). Vaccination with a multivalent Leptospira vaccine induces a protective immune response against the challenge of a homologous strain in hamsters, as well as a heterologous strain challenge for Leptospira Ballum (Gonzalez, M. et al., 2004 Biotecnologia Aplicada 21 (2004) 77-81).

Thus, as an alternative to vaccinating animals using all lepto serovar against which immunological protection is desired, it would be useful to provide new methods of eliciting cross-protective immune responses against different lepto serovars. Until the instant disclosure, methods for providing protection against LI copenhageni using non-copenhageni serovars were not known.

### SUMMARY OF THE INVENTION

The present invention provides a vaccine comprising an effective amount of non-copenhageni Leptospira serovar for use in providing a canine with protective immunity against Leptospira interrogans serovar copenhageni; wherein the vaccine is a multivalent/combination vaccine comprising the serovars Leptospira mterrogans (LI) icterohaemorrhagiae, LI canicola, LI grippotyphosa, and LI pomona.

A vaccine for use in providing protective immunity against a first Leptospira serovar comprising the step of administering a second Leptospira serovar(s), which is from a different serogroup and/or serovar, with respect to the first Leptospira serovar. In the cases where the second Leptospira serovar(s) is a combination of Leptospira serovar (e.g. a combination/multi-valent vaccine), the second Leptospira serovar(s) must not contain or comprise a Leptospira serovar of the same serovar as the first Leptospira serovar, for which protective immunity is being sought.

In an embodiment, the vaccine for use in the method of the invention provides protective immunity against Leptospira Icterohaemorrhagiae (LI) serovar copenhageni, and comprise the step of administering an effective amount of a non-copenhageni LI serovar to a canine animal in need thereof.

In a particular embodiment, the non-copenhageni LI serovar is from the 24^{th} passage of LI obtained from National Animal Disease Center (NADC), Ames, Iowa, on 28 February 1968 by Dow Chemical, Zionsville, Indiana.

In another embodiment, the vaccine for use in the method of the invention provides protective immunity against LI serovar copenhageni by administering a combination/multivalent Leptospira vaccine. In a particular embodiment, the vaccine is Merial's RECOMBITEK® 4 Lepto, as made in the United States as of June 25, 2012. In an embodiment, the 4 Lepto comprises *Leptospira canicolo, Leptospira grippotyphosa, Leptospira icterohaemorrhagiae, Leptospira pomona* (all chemically inactivated) and has label claims (as of June 25, 2012) according to the following: "provides protection against Leptospira grippotyphosa for 15 months and prevents shedding of Leptospira spirochetes in the urine. Specifically the vaccine is labeled to prevent Leptospirosis and Leptospiruria caused by L. icterohaemorrhagiae, L. canicola, L. grippotyphosa. It also aids in the prevention of Leptospirosis and Leptospiruria caused by L. Pomona."

In a particular embodiment, and unexpected/surprising to the skilled worker in possession of the current state-of-the-art knowledge in the field of leptospirosis, the invention provides a vaccine for use in a method of administration of Merial's RECOMBITEK® 4 Lepto to canines to elicit protective immunity against a first LI serovar, which is not contained within the 4-way vaccine, and which is LI serovar copenhageni.

These and other embodiments are disclosed or are obvious from and encompassed by, the following Detailed Description.

### BRIEF DESCRIPTION OF DRAWINGS

A full and enabling disclosure of the present invention, including the best mode thereof, to one of ordinary skill in the art, is set forth more particularly in the remainder of the specification, including reference to the accompanying figures, wherein:

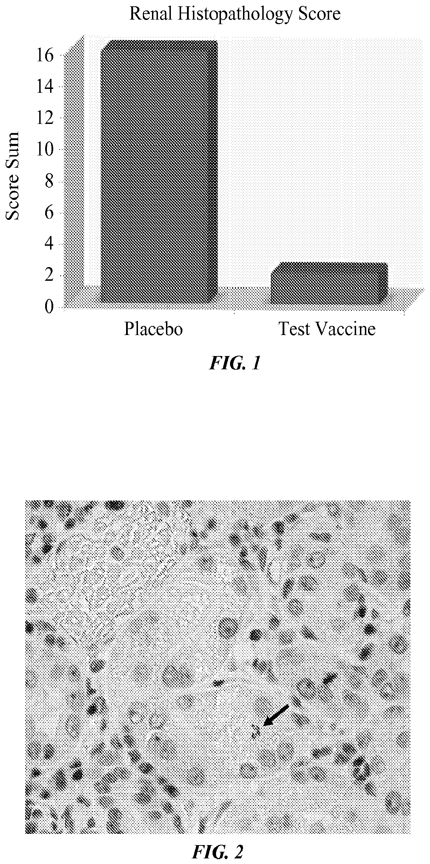

FIG. 1 provides a graph of renal histopathology scores by group;
FIG. 2 is an image of kidney immunohistochemistry (40X) from a control dog with acute leptospirosis. Arrow indicates the presence of leptospira spirochete in the renal tubules.

### DETAILED DESCRIPTION OF THE INVENTION

The present invention encompasses a vaccine for use in a method for prevention of infection due to Leptospires of a particular serovar by administering Leptospires of a different serovar.

In an embodiment, the invention provides methods of eliciting in a canine animal a protective immune response against Leptospira interrogans serovar copenhageni comprising the step of administering to the animal an effective amount of a non-copenhageni Leptospira serovar.

In an embodiment, the non-copenhageni Leptospira serovar is delivered as part of a multivalent/combination vaccine. In a particular embodiment, the non-copenhageni Lepto serovar is LI icterohaemorrhagiae.

In another embodiment, the vaccine comprises Leptospira Interrogans (LI) serovar icterohaemorrhagiae. In still another embodiment, the vaccine comprises LI icterohaemorrhagiae, LI canicola, LI grippotyphosa, and LI pomona.

In a particular embodiment, the vaccine is Merial's RECOMBITEK® 4 Lepto, as manufactured in June 2012.

### Descriptions/Definitions

The "LI serovar Ictero" seed from RECOMBITEK® 4 Lepto is identified herein as ictero, which was obtained from National Animal Disease Center (NADC), Ames, Iowa, on 28 February 1968 by Dow Chemical, Zionsville, Indiana. On 17 December 1971, the seed was transferred to Pitman-Moore, Inc., License No. 264, Washington Crossing, New Jersey. A master seed was prepared by Dow at the 20th passage. Pitman-Moore, Inc., produced a master seed after three passages in artificial medium on 21 January 1975 as "LI 1508 P23." A master seed was also qualified by Pitman-Moore, Inc., identified as "LI SC1518, P24 MS, 8/18/76." Rhone Merieux, Inc. (now known as Merial, Inc.), License No. 298, received a culture from Pitman-Moore, Inc., on 30 June 1988 identified as "LI SC1518 P24 MS 8/18/76".

The "LI serovar *copenhageni"* challenge strain was of different origin and serogroup/serovar, when compared to the vaccination serovars (isolated from a human in Brazil, and designated: Fiocruz L1-130).

By "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, an epitope, a hapten, or any combination thereof. Alternately, the immunogen or antigen may comprise a toxin or antitoxin.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

The term "immunogenic or antigenic polypeptide" as used herein includes polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the invention comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871; Geysen et al., 1984; Geysen et al., 1986. Similarly, conformational epitopes are readily identified by determining spatial conformation of amino acids such as by, e.g., x-ray crystallography and 2-dimensional nuclear magnetic resonance. See, e.g., Epitope Mapping Protocols, supra. Methods especially applicable to the proteins of T. parva are fully described in PCT/US2004/022605.

As discussed herein, the invention encompasses active fragments and variants of the antigenic polypeptide. Thus, the term "immunogenic or antigenic polypeptide" further contemplates deletions, additions and substitutions to the sequence, so long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cystine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms and/or clinical disease signs normally displayed by an infected host, a quicker recovery time and/or a lowered viral titer in the infected host.

By "animal" is intended mammals, birds, and the like. Animal or host as used herein includes mammals and human. The animal may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), ovine (e.g., sheep), bovine (e.g., cattle), porcine (e.g., pig), avian (e.g., chicken, duck, goose, turkey, quail, pheasant, parrot, finches, hawk, crow, ostrich, emu and cassowary), primate (e.g., prosimian, tarsier, monkey, gibbon, ape), ferrets, seals, and fish. The term "animal" also includes an individual animal in all stages of development, including newborn, embryonic and fetal stages.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

The invention will now be further described by way of the following non-limiting examples.

### EXAMPLES

### Example 1 - Cross-protection of Recombitek® 4 Lepto against a Leptospira interrogans serovar Copenhageni challenge in dogs

### Objective. To evaluate cross protection of RECOMBITEK® 4 Lepto against a Leptospira interrogans serovar Copenhageni challenge in dogs.

*Materials and Methods.* Twenty-four (24) purpose-bred beagles, approximately 2 months old, were randomly divided into two groups of 12 dogs each. One group was administered Recombitek® 4 Lepto and the other group a placebo vaccine (PBS). All dogs received 2 subcutaneous doses (1 ml) of the vaccine at a 21-day interval. Dogs from both groups were commingled during the entire study. Approximately 4 weeks after the second vaccination all dogs were sedated and administered *L. Copenhageni* challenge at 4.7 x 10⁸ lepto spirochetes/ml, 10 mls intraperitoneally and 0.2 ml instilled topically per eye in the conjunctival sac. Following challenge, blood was collected periodically for liver profile and leptospira re-isolation. Sera samples were tested for serovar specific antibody by microaglutination test at regular intervals. Urine was collected periodically for re-isolation of leptospira. Dogs were subject to necropsy at the end of the study and kidneys were harvested for histopathology and leptospira re-isolation.

**Table 1. Study Design**

| GROUP | DOGS PER GROUP | VACCINE GROUP | ROUTE/ DOSE | FREQUENCY | CHALLENGE* (29 Days post V2) |
|---|---|---|---|---|---|
| A | 12 | Recombitek® 4 Lepto | SC/1ml | Twice, 21 days apart | Conjunctival 0.2 ml / Intraperitoneal 10 ml |
| B | 12 | Placebo (PBS) | SC/1ml | Twice, 21 days apart | |

| | | | | | |
|---|---|---|---|---|---|
| *Challenge dose: 4.7 x 10⁸ organisms per ml, approximately 10^{9.73} organisms per dog. | | | | | |

*Results.* Dogs with mortality following *L.* Copenhageni challenge prior to planned necropsies were classified as having acute leptospirosis if one of the kidneys was positive for leptospirosis by immunohistochemistry and/or the histopathological lesions were indicative of acute leptospirosis. Dogs that underwent necropsy at the end of the study were classified as having disease due to *L. Copenhageni* if it had one or more positive urine samples and a renal histopathology score greater than or equal to 1 for either kidney.

**Table 2. Incidence of disease, leptospiuria and leptospiremia**

| *Group Name* | **(**-**)** | **(**+**)** |
|---|---|---|
| Incidence of disease due to *L. copenhageni* challenge | | |
| Placebo (PBS) | 1 | 10 |
| Test Vaccine (Recombitek® 4 Lepto) | 9 | 2 |
| p-value of Fisher's exact Test | P ≤ 0.01 | |
| Leptospiuria - presence of leptospira in the urine | | |
| Placebo (PBS) | 2 | 7 |
| Test Vaccine (Recombitek® 4 Lepto) | 8 | 1 |
| P-value of Fisher's exact Test | P ≤ 0.05 | |
| Leptospiremia - presence of leptospira in the blood | | |
| Placebo (PBS) | 1 | 10 |
| Test Vaccine (Recombitek® 4 Lepto) | 9 | 1 |
| p-value of Fisher's exact Test | P ≤ 0.01 | |

**Table 3. Clinical signs - incidence and duration post-challenge**

| ***Clinical Signs*** | ***Group*** | ***Total number of dogs*** | ***Mean duration (days)*** |
|---|---|---|---|
| Depression | Placebo (PBS) | 3/11 | 2.3 |
| | Recombitek® 4 Lepto | 0/11 | NA |
| Dehydration | Placebo (PBS) | 3/11 | 1 |
| | Recombitek® 4 Lepto | 1/11 | 1 |
| Icterus | Placebo (PBS) | 3/11 | 3 |
| | Recombitek® 4 Lepto | 0/11 | NA |
| Conjunctivitis | Placebo (PBS) | 9/11 | 15.4 |
| | Recombitek® 4 Lepto | 5/11 | 7.8 |

*Conclusions.* This is the first time we report cross protection of Recombitek® 4 Lepto against a *L. Copenhageni* challenge in dogs. These data are both surprising and unexpected in view of the overwhelming majority of literature references, which collectively teach vaccination with a particular leptospira species does not provide protective immune responses against heterologous leptospira species (see especially the "Leptospirosis Fact Sheet" (WHO, 2009) and Pena-Moctezuma, A. et al., 2001). The incidence of leptospirosis, and leptospira re-isolation from blood and urine was significantly higher in the placebo group in comparison to the Recombitek® Lepto 4 groups. Dogs in the placebo group had higher incidence of depression, dehydration, icterus and conjunctivitis. All dogs in the placebo group had a renal histopathology score of 1 or greater. Mean ALP, ALT, BUN and creatinine values on specific days were higher in the placebo group in comparison to the Recombitek® Lepto 4 group.

## Claims

1. A vaccine comprising an effective amount of non-copenhageni Leptospira serovars for use in providing a canine with protective immunity against Leptospira interrogans serovar copenhageni; wherein the vaccine is a multivalent/combination vaccine comprising the serovars Leptospira Interrogans (LI) icterohaemorrhagiae, LI canicola, LI grippotyphosa, and LI pomona.

2. The vaccine for use according to claim 1 wherein the serovars Leptospira Interrogans (LI) icterohaemorrhagiae, LI canicola, LI grippotyphosa, and LI pomona are chemically inactivated.

3. The vaccine for use according to claim 1 wherein about 1 ml of vaccine is to be administered to the canine.

4. The vaccine for use according to claim 1 wherein 2 subcutaneous doses are to be administered to the canine.

5. The vaccine for use according to claim 4 wherein the 2 doses are to be administered at a 21-day interval.

6. The vaccine for use according to claim 1 wherein the vaccine comprises additional antigens that provide immunity against additional canine pathogens.

7. The vaccine for use according to claim 6 wherein the additional antigens are selected from canine parvovirus (CPV), canine parainfluenza virus (CPi2), canine distemper virus (CDV), adenovirus, herpesvirus, rabies, canine coronavirus, and combinations thereof.

## Patentansprüche

1. Impfstoff, der eine wirksame Menge von Leptospira vom nicht-copenhageni Serotyp umfasst, zur Verwendung, um einem Hund schützende Immunität gegen Leptospira interrogans des Serotyps copenhageni zu verleihen, wobei der Impfstoff ein multivalenter Impfstoff/Kombinationsimpfstoff ist, der die Serotypen Leptospira Interrogans (LI) icterohaemorrhagiae, LI canicola, LI grippotyphosa und LI pomona umfasst.

2. Impfstoff zur Verwendung nach Anspruch 1, wobei die Serotypen Leptospira interrogans (LI) icterohaemorrhagiae, LI canicola, LI grippotyphosa und LI pomona chemisch inaktiviert sind.

3. Impfstoff zur Verwendung nach Anspruch 1, wobei dem Hund etwa 1 ml des Impfstoffes zu verabreichen ist.

4. Impfstoff zur Verwendung nach Anspruch 1, wobei dem Hund 2 subcutane Dosen zu verabreichen sind.

5. Impfstoff zur Verwendung nach Anspruch 4, wobei die 2 Dosen in einem Intervall von 21 Tagen zu verabreichen sind.

6. Impfstoff zur Verwendung nach Anspruch 1, wobei der Impfstoff zusätzliche Antigene umfasst, die Immunität gegen zusätzliche Hundepathogene verleihen.

7. Impfstoff zur Verwendung nach Anspruch 6, wobei die zusätzlichen Antigene ausgewählt sind aus Caninem Parvovirus (canine parvovirus; CPV), Caninem Parainfluenzavirus (canine parainfluenza virus; CPi2), Caninem Staupevirus (canine distemper virus; CDV), Adenovirus, Herpesvirus, Tollwut, Caninem Coronavirus (canine coronavirus), und Kombinationen davon.

## Revendications

1. Vaccin comprenant une quantité efficace de sérovars Leptospira non-copenhageni destiné à être utilisé pour conférer à un canin une immunité protectrice contre Leptospira interrogans sérovar copenhageni; où le vaccin est un vaccin multivalent/combiné comprenant les sérovars Leptospira Interrogans (LI) icterohaemorrhagiae, LI canicola, LI grippotyphosa et LI pomona.

2. Vaccin destiné à être utilisé selon la revendication 1 où les sérovars Leptospira Interrogans (LI) icterohaemorrhagiae, LI canicola, LI grippotyphosa et LI pomona sont inactivés chimiquement.

3. Vaccin destiné à être utilisé selon la revendication 1 où environ 1 ml de vaccin doit être administré au canin.

4. Vaccin destiné à être utilisé selon la revendication 1 où 2 doses sous-cutanées doivent être administrées au canin.

5. Vaccin destiné à être utilisé selon la revendication 4 où les 2 doses doivent être administrées à un intervalle de 21 jours.

6. Vaccin destiné à être utilisé selon la revendication 1 où le vaccin comprend des antigènes supplémentaires qui confèrent une immunité contre des pathogènes pour les canins supplémentaires.

7. Vaccin destiné à être utilisé selon la revendication 6 où les antigènes supplémentaires sont choisis parmi le parvovirus canin (CPV), le virus parainfluenza canin (CPi2), le virus de la maladie de Carré des canins (CDV), l'adénovirus, l'herpèsvirus, la rage, le coronavirus canin, et leurs combinaisons.
